# EUROPEAN PATENT APPLICATION

(11) **EP 0 958 806 A1**
(43) Date of publication of application: **24.11.1999**
(21) Application number: 98924557.6
(22) Date of filing: 10.06.1998
(51) Int. Cl.: A61K 7/13

(54) **HAIRCOLORING COMPOSITION**

(30) Priority: 11.06.1997 JP 16944697
(71) Applicant: NOVO NORDISK A/S, 2880 Bagsvaerd (DK)
(72) Inventor: YOSHINO, Teruhiko, Sumida-ku, Tokyo 130-8644 (JP); KIMURA, Asano, Sumida-ku, Tokyo 130-8644 (JP); ASAI, Yoshio, Sumida-ku, Tokyo 130-8644 (JP); YOSHIMOTO, Megumi, Sumida-ku, Tokyo 130-8644 (JP)
(86) International application number: JP9802555
(87) International publication number: WO9856335

(57) **Abstract**

A haircoloring composition characterized by containing a laccase and forming bubbles in its use. When applied to the hair, the composition is effective in dyeing the hair without lowering the effect of the laccase.

## Description

### TECHNICAL FIELD

The present invention relates to a hairdye composition capable of effectively dyeing hair of the head while causing little damage to the hair to which it has been applied.

### BACKGROUND OF THE INVENTION

An oxidation-type hairdye composition generally comprises an oxidation dye and an oxidizing agent, which are reacted with each other just before use and applied to hair to be dyed therewith. It may contain a color tone-adjusting agent of a so-called coupler component, which is for delicately adjusting the color of the dyed hair.

Heretofore, hydrogen peroxide has been essentially used as the oxidizing agent. However, it is known that hydrogen peroxide causes damage to hair, and most consumers are dissatisfied at their hair damaged thereby.

Many trials have heretofore been made for the purpose of reducing the damage to hair to be caused by hydrogen peroxide. As one trial, a technique of using oxidase in place of hydrogen peroxide has been proposed. Examples of the technique are known, which include using peroxidase as the oxidase (JP-A-47-10400, JP-A-53-32132), using laccase (U.S. Patent 3,251,742, JP-A-6-172145), using uricase (JP-A-63-246313), etc. However, of many such proposals, the case of using peroxidase requires adding hydrogen peroxide to the hairdye system owing to the characteristic of the enzyme, and the case of using uricase requires effective use of hydrogen peroxide to be formed through the enzymatic reaction for hairdyeing. Therefore, these cases could not basically solve the problem of how to prevent the damage by hydrogen peroxide to hair.

The case of using laccase does not require using hydrogen peroxide in the hairdye system, and is expected to cause little damage to hair. However, in laccase-containing hairdye compositions, the laccase could not react well to the intended degree owing to the fluidity and the liquid properties of the compositions. At present, therefore, laccase could not produce the expected results.

### DISCLOSURE OF THE INVENTION

Accordingly, the object of the invention is to provide a laccase-containing hairdye composition capable of effectively exhibiting the effect of laccase therein.

We, the present inventors have assiduously studied so as to attain the object noted above, and, as a result, have found that when a hairdye composition containing laccase along with a surfactant and/or a water-soluble polymer substance is foamed while being used, then it can effectively dye hair of the head without lowering the effect of laccase therein. On the basis of this finding, we have completed the present invention.

Accordingly, the invention provides a hairdye composition which contains laccase and which is foamed while it is used. In particular, it provides a hairdye composition that contains laccase and a developer along with a surfactant and/or a water-soluble polymer substance.

### BEST MODES OF CARRYING OUT THE INVENTION

The invention is described in detail hereinunder. The hairdye composition of the invention contains laccase as the oxidizing agent. Laccase used herein is an enzyme to be classified in E.C. 1.10.3.2. As one typical case of laccase reaction, known is oxidation of urushiol with laccase in Japanese lacquer to give a lacquer coat. Apart from Japanese lacquer, laccase exists in many plants and microorganisms, and this is an enzyme that catalyzes oxidation of aromatic compounds. Any and every laccase is usable herein irrespective of its sources.

The amount of laccase to be in the composition of the invention varies, depending on the form of the composition, the frequency of using the composition, the time for treatment with the composition, and the titer value of the enzyme, but may generally fall between 0.0005 and 1 % by weight (hereinafter % is all by weight), preferably between 0.005 and 0.5 %. If its amount is smaller than 0.0005 %, laccase could not exhibit its effect satisfactorily. However, even if its amount is larger than 1 %, the increase in the amount could not bring about any more increased effect of laccase.

The developer for use in the invention may be freely selected from any components capable of coloring in the presence of laccase. As examples of the compounds, mentioned are oxidation dyes such as typically paraphenylenediamine, para-aminophenol, toluene-2,5-diamine, toluene-3,4-diamine, etc. Salts of those compounds with hydrochloric acid, sulfuric acid or acetic acid are also usable herein. Apart from ordinary oxidation dyes such as those mentioned above, also usable are other compounds capable of coloring in the presence of laccase, such as 3,4-diaminobenzohydrazide, 3,5-diaminobenzohydrazide, 3-hydroxytyramine, catechin, etc. Ordinary oxidation dyes have sensitizability and may have some negative influences on human bodies, but those except oxidation dyes have no sensitizability and are therefore especially effective in view of their safety.

The composition of the invention may contain a coupler component, with which the composition could attain delicate color differences which could not be attained by the single use of a developer only. Though depending on the developer used, any component usable in ordinary oxidation-type hairdye compositions is employable herein as the coupler.

The amount of the developer and that of the coupler to be in the composition of the invention vary, depending on the frequency of using the composition and the form of the composition, but may generally fall between 0.01 and 10 %, preferably between 0.1 and 5 %.

The hairdye composition of the invention preferably contains a surfactant. As the surfactant, preferably used at least one selected from nonionic surfactants, especially polyoxyethylene alkyl ethers and fatty acid alkylolamides, cationic surfactants, and carboxylate-type anionic surfactants, or their combinations.

Specific examples of the surfactant are mentioned below. Examples of polyoxyethylene alkyl ethers include polyoxyethylene stearyl ether, polyoxyethylene-hardened castor oil, etc.; examples of fatty acid alkylolamides include coconut oil fatty acid diethanolamide, etc.; examples of cationic surfactants include stearylmethylammonium chloride, distearyldimethylammonium chloride, etc.

Carboxylate-type anionic surfactants include fatty acid soaps, such as potassium, sodium, triethanolamine or ammonium salts of C₁₂₋₁₈ saturated or unsaturated fatty acids and their mixtures of coconut oil fatty acid, hardened coconut oil fatty acid, palm oil fatty acid, hardened palm oil fatty acid, beef tallow fatty acid, hardened beef tallow fatty acid, etc.; as well as alkyl ether carboxylate salts, N-acylsarcosine salts, N-acyl-β-alanine salts, N-acylglutamate salts, etc. Of those, preferred are N-acylglutamate salts. Specific examples of N-acylglutamate-type surfactants include N-cocoyl-L-glutamate triethanolamine, lauroyl-L-glutamate triethanolamine, sodium N-cocoyl-L-glutamate, sodium N-lauroyl-L-glutamate, sodium N-myristoyl-L-glutamate, sodium N-cocoyl,hardened talloyl-L-glutamate, potassium N-cocoyl-L-glutamate, etc.

The amount of the surfactant to be in the composition of the invention generally falls between 0.05 and 5.0 %, but preferably between 0.1 and 2.0 %.

The hairdye composition of the invention may contain a water-soluble polymer substance. The water-soluble polymer substance to be used herein may be any one that is generally used in ordinary cosmetics, but preferred is at least one selected from cationated cellulose, hydroxyethyl cellulose, hydroxymethyl cellulose, hydroxypropylmethyl cellulose, amphoteric resins (Yukaformers), vinyl ethyl ether/maleate polymer, polyvinylpyrrolidone, acrylic resin alkanolamines and the like, or their combinations.

The amount of the water-soluble polymer substance to be in the composition of the invention generally falls between 0.01 and 10.0 %, but preferably between 0.05 and 5.0 %.

After having been prepared, the hairdye composition of the invention is generally in liquid, and this is foamed while being used. For foaming the composition, any known means is employable. For example, employable is a method of passing the composition through a mesh in the container that contains the composition, to thereby make the composition foamy; or a method of charging the composition in a pressure container along with any ordinary propellant, carbon dioxide, LPG or the like, and discharging it from the container under pressure to thereby make the composition foamy.

The composition of the invention may additionally contain any other ingredients that are generally used in ordinary cosmetics, such as oily components, moisturizers, lower alcohols, thickeners, antioxidants, chelating agents, softeners, pH-adjusting agents, preservatives, fragrances, etc., in addition to the components mentioned above.

According to the invention, there is provided a hairdye composition capable of effectively dyeing hair to which the composition has been applied, without lowering the effect of laccase therein.

The invention is described concretely with reference to the following Examples and Comparative Examples, which, however, are not intended to restrict the scope of the invention.

### [Example 1, Comparative Examples 1 and 2]

Different types of hairdye compositions were prepared in an ordinary manner, and tested for their hairdyeing effect. Precisely, the hairdye compositions mentioned below were prepared, and one g of each composition was applied to human gray hair having a weight of 0.5 g and a length of 10 cm, and left at 30°C for 30 minutes.

After having been thus left, the gray hair was taken out, washed with water, shampooed, and dried in air. Then, its color difference (ΔE) was measured with a color-difference meter. Based on the data as the index for its hairdyeing effect, each composition was evaluated according to the criteria mentioned below. In addition, the dyed hair was evaluated for its outward appearance, according to the criteria mentioned below. The results are shown in Table 1.

### 〈Criteria for Hairdyeing Effect of Composition〉

- ⊙⃝:: The difference in ΔE between the non-treated hair and the dyed hair fell between 30 and 40.
- ○ :: The difference in ΔE between the non-treated hair and the dyed hair fell between 20 and 30.
- △:: The difference in ΔE between the non-treated hair and the dyed hair fell between 10 and 20
- X:: The difference in ΔE between the non-treated hair and the dyed hair fell between 0 and 10.

### 〈Criteria for Appearance of Dyed Hair〉

- ○ :: Dyed uniformly.
- △:: Dyed somewhat unevenly.
- X:: Dyed extremely unevenly.

### Composition 1: Example 1 (foam composition)

| | Amount (wt.%) |
|---|---|
| Paraphenylenediamine | 0.1 |
| Laccase | 0.1 |
| Cationated Cellulose | 3.0 |
| Polyoxyethylene(40)-Hardened Castor Oil | 1.0 |
| Ethyl Alcohol | 10.0 |
| Phosphate Buffer (0.1 M, pH 6) | 5.0 |
| Pure Water | balance |
| Total | 100.0 |

After having been prepared, the composition was charged into a container provided with a mesh therein, and discharged therefrom through the mesh to test the foamy composition.

### Composition 2: Comparative Example 1 (cream composition)

| | Amount (wt.%) |
|---|---|
| Paraphenylenediamine | 0.1 |
| Laccase | 0.1 |
| Liquid Paraffin | 15.0 |
| Vaseline | 15.0 |
| Polyoxyethylene Nonyl Ethyl Ether | 5.0 |
| Glycerin | 5.0 |
| Phosphate Buffer (0.1 M, pH 6) | 5.0 |
| Pure Water | balance |
| Total | 100.0 |

### Composition 3: Comparative Example 2 (gel composition)

| | Amount (wt.%) |
|---|---|
| Paraphenylenediamine | 0.1 |
| Laccase | 0.1 |
| Hydroxyethyl Cellulose | 1.0 |
| Polyvinylpyrrolidone | 2.0 |
| Phosphate Buffer (0.1 M, pH 6) | 5.0 |
| Pure Water | balance |
| Total | 100.0 |

**Table 1**

| | Example 1 (foam composition) | Comparative Example 1 (cream composition) | Comparative Example 2 (gel composition) |
|---|---|---|---|
| Hairdyeing Effect of Composition | ⊙⃝ | △ | ○ |
| Appearance of Dyed Hair | ○ | △ | △ |

As is obvious from Table 1, it is known that the foam composition of the invention effectively exhibits the ability of laccase therein and displays the hairdyeing effect intrinsic to it, but the other compositions could not exhibit the ability of laccase therein. Regarding the appearance of the dyed hair, it is known that the coloration of the hair dyed with the cream or gel composition is uneven in some degree but that of the hair dyed with the foam composition is even.

### [Example 2]

A hair mousse composition comprised of the components mentioned below was prepared, and applied to hair, while being foamed along with liquefied petroleum gas (mousse/liquefied petroleum gas = 9/1), to evaluate it for its hairdyeing effect. The hair as dyed with the composition showed good and uniform dark brown color tone, and the hairdyeing effect of the composition was good.

| | Amount (wt.%) |
|---|---|
| Toluene-2,5-diamine | 2.0 |
| Para-aminophenol | 1.0 |
| Meta-aminophenol | 0.03 |
| Laccase | 0.3 |
| Methyl-polysiloxane (weight-average molecular weight: 100,000) | 4.5 |
| Methyl-polysiloxane (30 cs) | 2.5 |
| N-cocoyl-L-glutamate Triethanolamine | 0.5 |
| Yukaformer 301 | 0.9 |
| Ethanol | 5.0 |
| Pure Water | balance |
| Total | 100.0 |

### [Example 3]

A hair foam composition comprised of the components mentioned below was prepared, and applied to hair, while being foamed along with liquefied petroleum gas (foam/liquefied petroleum gas = 9/1), to evaluate it for its hairdyeing effect. The hair as dyed with the composition showed good and uniform black color tone, and the hairdyeing effect of the composition was good.

| | Amount (wt.%) |
|---|---|
| Paraphenylenediamine | 0.3 |
| Laccase | 0.01 |
| Acrylic Resin Alkanolamine (50 %) | 4.0 |
| Stearylmethylammonium Chloride | 0.5 |
| Polyoxyethylene Stearyl Ether | 2.0 |
| Liquid Paraffin | 4.5 |
| Ethanol | 15.0 |
| Glycerin | 5.0 |
| Pure Water | balance |
| Total | 100.0 |

## Claims

1. A laccase-containing hairdye composition, which is foamed while it is used.

2. The hairdye composition as claimed in claim 1, which contains a developer along with a surfactant and/or a water-soluble polymer substance.
